# EUROPEAN PATENT SPECIFICATION

(11) **EP 1 565 609 B1**
(45) Date of publication and mention of the grant of the patent: **27.09.2006**
(21) Application number: 03811838.6
(22) Date of filing: 10.11.2003
(51) Int. Cl.: D06M 23/12, D06M 17/00, B01J 13/02, C09J 7/04, A41D 31/00, A61K 8/00

(54) **FLEXIBLE MATERIAL INCLUDING CONTROLLED SUBSTANCE RELEASE**
FLEXIBLES MATERIAL MIT KONTROLLIERTER WIRKSTOFFFREIGABE
MATERIAU SOUPLE A LIBERATION CONTROLEE DE SUBSTANCE

(30) Priority: 22.11.2002 US 428461 P
(43) Date of publication of application: 24.08.2005
(73) Proprietor: Koninklijke Philips Electronics N.V., 5621 BA Eindhoven (NL)
(72) Inventor: MARMAROPOULOS, George, Briarcliff Manor, NY 10510-8001 (US)
(74) Representative: White, Andrew Gordon
(86) International application number: PCT/IB2003/005309
(87) International publication number: WO 2004/048678

(56) References cited:
- EP-A- 0 328 937
- EP-A- 0 480 162
- EP-A- 1 054 095
- US-A- 4 514 461
- "Fiber cloth for dress shirt, underwear or leg knitted fabric, includes microcapsules which comprise organic resin as wall material and contain mentha oil and/or 1-menthol" DERWENT, XP002219312
- NELSON G: "MICROENCAPSULATES IN TEXTILE COLORATION AND FINISHING" REVIEW OF PROGRESS IN COLORATION & RELATED TOPICS, SOCIETY OF DYERS AND COLOURISTS. BRADFORD, GB, vol. 21, 1991, pages 72-85, XP000225719 ISSN: 0557-9325

## Description

The invention relates to wearable electronics and the controlled release of substances.

Microencapsulation technology involves encapsulating small amounts of substance, such as perfume, in plastic, gelatin, or polymer spheres that are a few microns in diameter. The spheres release the substance upon rupturing. Rupturing occurs either mechanically (i.e. by scratching with a fingernail or pulling apart adhesives on either side of the spheres), chemically (i.e., by dissolving the spheres in a solvent to enable release of the substance), or thermally (i.e., by heating the spheres with an external source above their melting points to initiate rupturing). A variety of substances can be encapsulated in microcapsules. As an example, the substance can be a fragrance, a dye, a lotion, or oil. Substances can be contained in microcapsules for as long as several years.

Conventionally, use of microcapsules has been limited to scent inserts in magazines and commercially available "scratch-and-sniff" merchandise, both of which are relatively inflexible.

United States patent number 4,990,392 (the "'392 patent"), describes a textile product that includes microcapsules applied to the fabric using thermo-adhesive materials. The '392 patent describes adding microcapsules containing substances to a textile such that the microcapsules will withstand washing and other normal uses of the textile. The microcapsules rupture during ordinary use of the textile either due to a rise in temperature or an increase in pressure. However, the release of substances contained in the microcapsules cannot be selectively controlled.

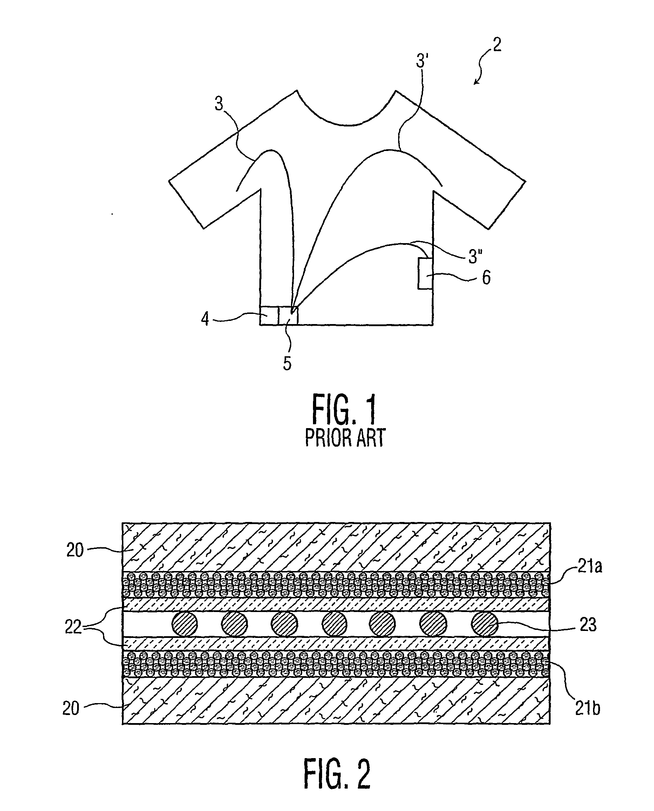

Fabrics including conductive fibers are also commonly known in the art. Such fabrics include fibers interwoven with textile fibers to create circuits. Current can be selectively passed to an area on such fabric using a switch and a power source. Fig. 1 depicts a garment 2 that includes conductive fibers. Garment 2 has three current paths 3, 3', 3" that are made up of conductive fibers through which current can be passed from power source 4. Current paths can also be formed using other known techniques such as conductive ink. Garment 2 also includes a switch 5 that the wearer of the garment can select which current path he or she chooses. For example, a user can attach a portable electronic device, such as a cellular telephone or portable radio, to garment 2 at clip 6. When the wearer sets switch 5 to power the electronic device, current passes from power source 4 through current path 3" to clip 6 and into the attached device. The conductive fibers that make up current paths 3, 3', and 3" can also be selected to have a high resistance. Consequently, they act as resistors and release electrical energy as heat. Current paths 3, 3', and 3" can be used to heat garment 2 in selected areas. However, these fabrics are not used to selectively control the release of a substance from within the fabric itself.

Accordingly, it would be desirable for a flexible fabric to include a means for selectively controlling the release of a substance that does not suffer from the prior art limitations.

A flexible material including several elements meets the need for a flexible material that allows for the controlled release of a microencapsulated substance in one aspect. One element is interwoven fibers. A second element is means for passing a current and generating localized heating interspersed among the fibers. A third element is at least one microcapsule, situated on or within the interwoven fibers and means for passing a current, containing a substance and releasing said substance upon rupture due to localized heating generated by selectively heating the means for passing a current. A fourth element is a means for controlling the current passed through the means for passing a current to enable controlled localized heating.

In one embodiment, the means for passing a current and generating localized heating is conductive fibers. In another embodiment, the means for passing a current and generating localized heating is conductive ink.

In yet another embodiment, the at least one microcapsule further comprises a thermo-plastic polymer. In another embodiment, the thermo-plastic polymer forms the at least one microcapsule containing the substance to be released.

In yet another embodiment, the at least one microcapsule releases the substance upon reaching its melting point. In another embodiment, the substance has a lower vapor point than the melting point of the at least one microcapsule.

In yet another embodiment, the substance is an oil, liquid, or solid material. In another embodiment, the substance generates a scent upon release into the ambient environment.

In still another embodiment, the means for controlling the current includes a power source and a current path selector. In another embodiment, the means for controlling the current further includes at least one programmable sensor which determines when to activate and deactivate the means for passing a current. In another embodiment, the at least one programmable sensor senses when a predetermined number of microcapsules have ruptured. The means for controlling the current can also include a timer. In one embodiment the timer determines when to deactivate the means for passing a current based upon the melting point of the at least one microcapsule, the number of microcapsules, and the material properties of the substance contained in the at least one microcapsule.

In another embodiment, the flexible material includes multiple microcapsules a first portion of which contain a first substance and a second portion of which contain a second substance. In another embodiment, the first substance and the second substance have different material properties. The different material properties can be scent, melting point, viscosity, physical state, color, flavor, chemical composition, and texture. In another embodiment, the first portion of microcapsules containing the first substance are grouped on or within an area of the flexible material such that the means for controlling the current locally heats the area and enables the release of the first substance. In another embodiment, the means for controlling the current allows local heating of either the first or the second portion of microcapsules and controllably enables the release of the first or second substance.

In still another embodiment, the means for controlling the current allows local heating of both the first and the second portion of microcapsules and controllably enables the release of a portion of the first and second substances.

In another embodiment, the first portion of microcapsules has a different melting point than the second portion of microcapsules.

In another embodiment, the means for controlling the current includes means for locally heating the first and second portion of microcapsules such that only the first portion of microcapsules rupture and release the substance they contain.

In one aspect a flexible material includes interwoven fibers; means for passing a current and generating localized heating interspersed among the fibers; at least one substance, situated on or within the fibers and means for passing a current, that vaporizes due to localized heating generated by selectively heating the means for passing a current; and means for controlling the current passed through the means for generating localized heating.

In another aspect of the invention, a method of controllably releasing a substance contained in a flexible material includes several steps. One step is integrating fibers and means for passing a current and generating localized heating interspersed among the textile fibers. Another step is forming at least one microcapsule containing a substance. Another step is incorporating the at least one microcapsule above or within the integrated fibers and means for passing a current. Another step is selectively heating the at least one microcapsule. Another step is rupturing the at least one microcapsule, and another step is releasing the substance.

In another aspect of the invention, a method of controllably releasing a substance contained in a flexible material includes integrating fibers and means for passing a current and generating localized heating interspersed among the textile fibers; forming a substance above or within the interwoven textile fibers and means for passing a current; selectively heating the substance; and evaporating the substance.

The invention provides many advantages that are evident from the following description, drawings, and claims.

The invention may be more completely understood in reference to the following figures:
Fig. 1 depicts a prior art garment including conductive fibers;
Fig. 2 depicts a cross section of a flexible material including conductive fibers and microencapsulated substances;
Fig. 3 depicts a garment including conductive fibers and multiple zones of microencapsulated substances;
Fig. 4 depicts a cross section of an area on a flexible conductive material with microencapsulated substances in separate zones on its surface; and
Fig. 5 depicts another embodiment of a flexible conductive material with microencapsulated substances in separate zones on its surface.

Fig. 2 depicts a cross section of a flexible material including conductive fibers and microencapsulated substances. The flexible material includes conductive fibers 23 made of a conductive material and woven into textile fiber layers 20. Conductive fibers 23 are covered on both sides by insulating layers 22 which prevent microcapsule layers 21a and 21b from overheating or heating too quickly. Layers 21a and 21b are composed of microcapsules containing a substance, for example perfume. When current is passed through conductive fibers 23, they act as resistors and give off a fixed amount of heat. This heats insulating layers 22 and microcapsule layers 21a and 21b. As individual microcapsules begin to heat, the perfume they contain reaches its vapor point. As individual microcapsules reach their melting points, they rupture and release the vaporized perfume.

Insulating layers 22 can be selected to ensure that only a limited amount of heat will reach the microcapsules so that a limited number of microcapsules will break each time a user passes a current through conductive fibers 23. This allows the flexible material to be used multiple times since only a few microcapsules are broken during each usage. The insulating layers can also exist as coatings surrounding conductive fibers 23. Additionally, the amount of current passed through conductive fibers 23 can also be set to achieve the desired amount of microcapsule breakage. The desired number of microcapsules to be broken during a single usage depends upon the material properties of the substances they contain. For example, if the microcapsules contain a concentrated strong fragrance, only a few need be broken in a single use.

In Fig. 2, microcapsule layers 21a and 21b can also be composed of microcapsules containing different substances. For exemplary purposes, assume that the flexible material is part of a garment. As such, layer 21a is closer to the ambient environment and layer 21b is closer to the skin of the wearer. The microcapsules in layer 21a can contain perfume, since the substance of perfume should have sufficient exposure to the ambient environment. The microcapsules in layer 21b can contain skin lotion, since the release of lotion should occur closer to the wearer's skin.

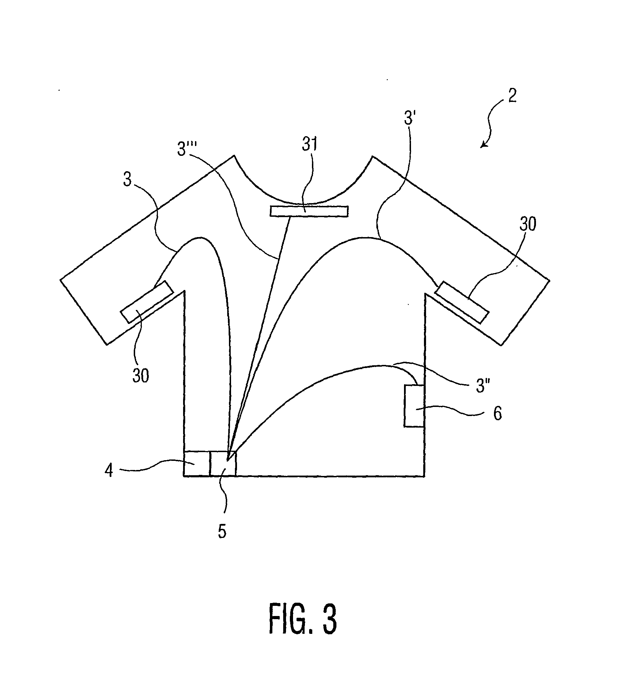

Fig. 3 depicts a garment 2 made of a flexible material that includes conductive fibers and multiple zones of microencapsulated substances. Here a user can set controller 50 to either activate zones 30, zone 31 or to power device clip 6 from power source 4. Zones 30 contain, for example, microencapsulated deodorizing antiperspirant. Since these zones are situated in proximity to a user's armpits, the user can activate these zones such that the microcapsules therein are ruptured and provide for the release of deodorizing antiperspirant. In addition, zones 30 can contain sensors which signal controller 50 to generate current in zones 30 when they sense deodorizing or perspiring occurs. The sensors can also dictate when controller 50 should deactivate zones 30, i.e., when a certain amount of microcapsules have ruptured, or when the sensors no longer detect order or perspiring. Further, controller 50 can also be programmed to shut off current to zones 30 automatically after a preset period of time determined by the melting point and/or the number of microcapsules in zone 30. The sensors can be any type of biosensor, chemical sensor, or mechanical sensor known in the art.

Zone 31 contains, for example, at least one fragrance. If the wearer of garment 2 wishes to alter the scent of the ambient environment, he or she can set controller 50 to activate zone 31. Activation will pass current from power source 4 through current path 3"' to zone 31. The conductive fibers in zone 31 heat up such that a portion of the microcapsules in zone 31 rupture and release fragrance. Controller 50 can automatically shut off current to zone 31 after a preset period of time based on the melting point of the microcapsules in zone 31 as well as the material properties of the substance contained in the microcapsules. For example, controller 50 can be programmed to generate current for 30 seconds to zone 31 because the rate at which the microcapsules in zone 30 rupture would release ample fragrance to comfortably alter the user's immediate environment in that period of time. Further, a user can manually set controller 50 based on their own personal preference.

Garment 2 is manufactured such that the microcapsules and conductive fibers in zones 30 and 31, as well as conductive fibers in current paths 30, 30', 30", and 30"' can be washed with other garments without breaking any microcapsules or affecting any conductive fibers. In addition, garment 2 can include enough microcapsules in a given zone to allow for a certain number of uses. As an example, zone 31 can contain enough microcapsules that the fragrance contained therein can be released 600 times at 30 seconds of current per activation. Thus, garment 2 has a defined useful life determined based upon the number of microcapsules in an area, the rate at which they rupture, the amount of time they are heated, and the melting point of the microcapsules. A further advantage is that even when garment 2 achieves its useful life in terms of zones 30 and 31, it can still be worn as a garment. Further, Garment 2 can still support portable electronic devices by passing current from power source 4 through current path 3" to clip 6 onto which a device can be attached and powered. Garment 2 can be any type of garment worn by a user and is not limited to the shirt depicted in Fig. 3. Further, any type of flexible material can include zones such as zones 30 and 31.

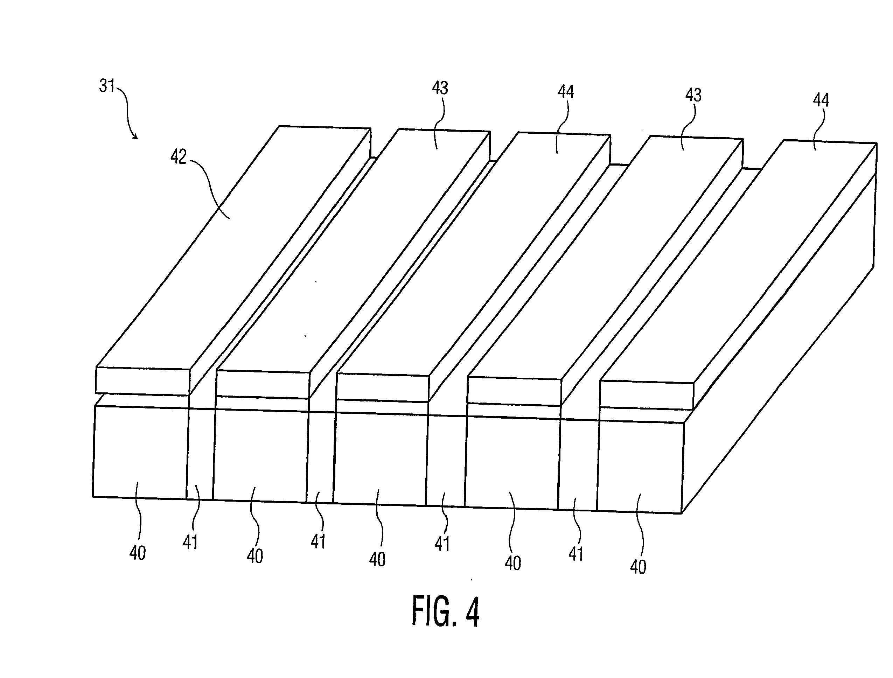

Fig. 4 depicts a cross section of a layer of flexible material, for example, zone 31. In Fig. 4, strips of different microencapsulated substances 42, 43, 44 are deposited above conductive areas 40. As an example, a user can set controller 50 to generate a fragrance that is composed of 2 parts substance 44 and one part substance 42. Controller 50 passes current through current paths that heat up the conductive fabric areas 40 under strips corresponding to microencapsulated substances 42, 44, 44. Insulators 41 prevent adjoining strips from being heated. Thus within a given zone, multiple substances can be simultaneously and controllably released.

Controller 50 can be programmed with the relative proportions of each strip needed to produce a given result, such as a given coloration or fragrance. Thus, when a user selects the given coloration or fragrance, controller 50 will automatically pass current from power source 4 through the appropriate current path to heat the appropriate conductive areas 40 to cause the requisite proportions of substance to be released due to microcapsule rupture.

In addition, strips corresponding to substances 42, 43, 44 can be formed from microcapsules with different melting points for each different substance 42, 43, 44. Thus, conductive areas 40 can all be heated simultaneously and based on the different melting points of the microcapsules, different proportion of substances 42, 43, 44 can be released.

Depositing substance directly onto zone 31 can also form strips corresponding to substances 42, 43, 44. In other words, conductive areas 40 can selectively heat substances 42, 43, 44 to either liquefy, evaporate, or sublimate and cause the same result as if substances 42, 43, 44 were microencapsulated.

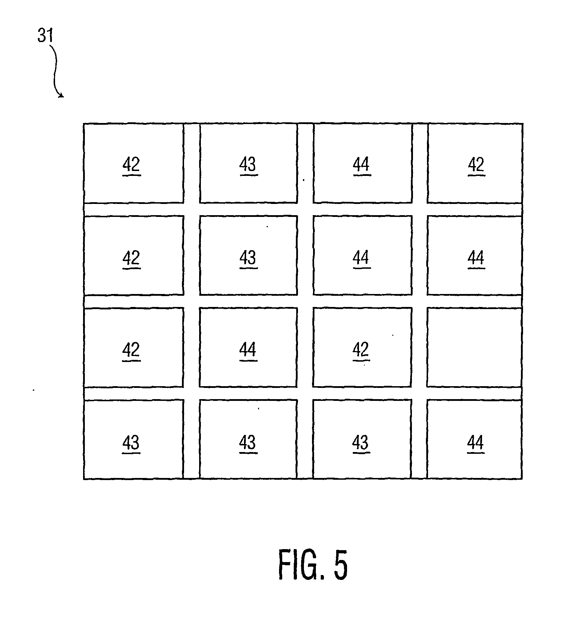

Fig. 5 depicts a matrix arrangement of zone 31, whereby controller 50 can selectively activate sub-zones 42, 43, 44 according to the user's desired effect. Each sub-zone 42, 43, 44 is independently controllable such that controller 50 can pass current from power source 4 through a current path to a conducive area 40 below a given sub-zone and heat only the desired sub-zone.

## Claims

1. A flexible material comprising:
interwoven fibers (20);
means for passing a current (23) and generating localized heating interspersed among the fibers (20);
at least one microcapsule, situated on or within the interwoven fibers (20) and means for passing a current (23), containing a substance and releasing said substance upon rupture due to localized heating generated by selectively heating the means for passing a current (23); and
means for controlling the current (5) passed through the means for passing a current (23) to enable controlled localized heating.

2. The flexible material of Claim 1, wherein the means for passing a current (23) and generating localized heating further comprises conductive fibers.

3. The flexible material of Claim 1, wherein the means for passing a current (23) and generating localized heating further comprises conductive ink.

4. The flexible material of Claim 1, wherein the at least one microcapsule further comprises a thermo-plastic polymer.

5. The flexible material of Claim 4, wherein the thermo-plastic polymer forms the at least one microcapsule containing the substance to be released.

6. The flexible material of Claim 1, wherein the at least one microcapsule releases the substance upon reaching its melting point.

7. The flexible material of Claim 6, wherein the substance has a lower vapor point than the melting point of the at least one microcapsule.

8. The flexible material of Claim 1, wherein the substance consists of at least one of oil, liquid, and solid material.

9. The flexible material of Claim 1, wherein the substance generates a scent upon release into the ambient environment.

10. The flexible material of Claim 1, wherein the means for controlling the current (5) further comprises a power source (4), and a current path selector (5).

11. The flexible material of Claim 10, wherein the means for controlling the current (5) further comprises at least one programmable sensor which determines when to activate and deactivate the means for passing a current.

12. The flexible material of Claim 11, wherein the at least one programmable sensor senses when a predetermined number of microcapsules have ruptured.

13. The flexible material of Claim 10, wherein the means for controlling the current further comprises a timer.

14. The flexible material of Claim 13, wherein the timer determines when to deactivate the means for passing a current (23) based upon the melting point of the at least one microcapsule, the number of microcapsules, and the material properties of the substance contained in the at least one microcapsule.

15. The flexible material of Claim 1 further comprising multiple microcapsules wherein a first portion (42) of the microcapsules contain a first substance and a second portion (43) of the microcapsules contain a second substance.

16. The flexible material of Claim 15, wherein the first substance and the second substance have different material properties.

17. The flexible material of Claim 16, wherein the different material properties consist of at least one of scent, melting-point, viscosity, physical state, color, flavor, chemical composition, and texture.

18. The flexible material of Claim 15, wherein the first portion of microcapsules (42) containing the first substance are grouped on or within an area of the flexible material such that the means for controlling the current locally heats the area and enables the release of the first substance.

19. The flexible material of Claim 16, wherein the means for controlling the current (5) allows local heating of either the first (42) or the second (43) portion of microcapsules and controllably enables the release of the first or second substance.

20. The flexible material of Claim 16, wherein the means for controlling the current (5) allows local heating of both the first (42) and the second (43) portion of microcapsules and controllably enables the release of a portion of the first and second substances.

21. The flexible material of Claim 15, wherein the first portion of microcapsules (42) has a different melting point than the second portion (43) of microcapsules.

22. The flexible material of Claim 21, wherein the means for controlling the current (5) further comprises means for locally heating the first (42) and second (43) portion of microcapsules such that only the first portion (42) of microcapsules rupture and release the substance they contain.

23. A flexible material comprising:
interwoven fibers (20);
means for passing a current (23) and generating localized heating interspersed among the fibers (20);
at least one substance, situated on or within the fibers and means for passing a current, that vaporizes due to localized heating generated by selectively heating the means for passing a current (23); and
means for controlling the current (5) passed through the means for generating localized heating.

24. A method of controllably releasing a substance contained in a flexible material comprising:
integrating fibers (20) and means for passing a current (23) and generating localized heating interspersed among the fibers (20);
forming at least one microcapsule containing a substance;
incorporating the at least one microcapsule above or within the integrated fibers (20) and means for passing a current (23);
selectively heating the at least one microcapsule;
rupturing the at least one microcapsule; and
releasing said substance.

25. A method of controllably releasing a substance contained in a flexible material comprising:
integrating fibers (20) and means for passing a current (23) and generating localized heating interspersed among the fibers (20);
forming a substance above or within the integrated fibers (20) and means for passing a current (23) ;
selectively heating the substance; and
evaporating said substance.

## Patentansprüche

1. Flexibles Material, das die nachfolgenden Elemente umfasst:
- verflochtene Fasern (20);
- Mittel zum Zuführen eines Stromes (23) und zum Erzeugen örtlicher Erhitzung zwischen den Fasern (20);
- wenigstens eine Mikrokapsel, die sich auf oder in den verflochtenen Fasern (20) und den Mitteln zum Zuführen eines Stromes (23) befindet, mit einer Substanz, wobei die genannte Substanz bei Bruch freigegeben wird, verursacht durch örtliche Erhitzung, erzeugt durch eine selektive Erhitzung der Mittel zum Zuführen eines Stromes (23); und
- Mittel zur Steuerung des Stromes (5) durch die Mittel zum Zuführen eines Stromes (23) zum Ermöglichen einer kontrollierten örtlichen Erhitzung.

2. Flexibles Material nach Anspruch 1, wobei die Mittel zum Zuführen eines Stromes (23) und zu Erzeugen einer örtlichen Erhitzung weiterhin leitende Fasern aufweisen.

3. Flexibles Material nach Anspruch 1, wobei die Mittel zum Zuführen eines Stromes (23) und zum Erzeugen einer örtlichen Erhitzung weiterhin leitende Tinte aufweisen.

4. Flexibles Material nach Anspruch 1, wobei die wenigstens eine Mikrokapsel weiterhin ein thermoplastisches Polymer aufweist.

5. Flexibles Material nach Anspruch 4, wobei das thermoplastische Polymer die wenigstens eine Mikrokapsel bildet, welche die freizugebende Substanz enthält.

6. Flexibles Material nach Anspruch 1, wobei die wenigstens eine Mikrokapsel beim Erreichen des Schmelzpunktes die Substanz freigibt.

7. Flexibles Material nach Anspruch 6, wobei die Substanz einen niedrigeren Verdampfungspunkt hat als der Schmelzpunkt der wenigstens einen Mikrokapsel.

8. Flexibles Material nach Anspruch 1, wobei die Substanz aus wenigstens einem Element aus Öl, Flüssigkeit und Feststoff besteht.

9. Flexibles Material nach Anspruch 1, wobei die Substanz bei Freigabe in die Umgebung einen Duft erzeugt.

10. Flexibles Material nach Anspruch 1, wobei die Mittel zur Steuerung des Stromes (5) weiterhin eine Speisequelle (4) sowie einen Stromstreckenselektor (5) aufweisen.

11. Flexibles Material nach Anspruch 10, wobei die Mittel zur Steuerung des Stromes (5) weiterhin wenigstens einen programmierbaren Sensor aufweisen, der bestimmt, wann die Mittel zum Zuführen eines Stromes aktiviert und deaktiviert werden sollen.

12. Flexibles Material nach Anspruch 11, wobei der wenigstens eine programmierbare Sensor fühlt, wann eine vorbestimmte Anzahl Mikrokapseln zerrissen worden sind.

13. Flexibles Material nach Anspruch 10, wobei die Mittel zur Steuerung des Stromes weiterhin einen Zeitgeber enthalten.

14. Flexibles Material nach Anspruch 13, wobei der Zeitgeber bestimmt, wann die Mittel zum Zuführen eines Stromes (23) auf Basis des Schmelzpunktes der wenigstens einen Mikrokapsel, der Anzahl Mikrokapseln und der Materialeigenschaften der in der wenigstens einen Mikrokapsel vorhandenen Substanz deaktiviert werden sollen.

15. Flexibles Material nach Anspruch 1, das weiterhin mehrere Mikrokapseln aufweist, wobei ein erster Teil (42) der Mikrokapseln eine erste Substanz enthält und ein zweiter Teil (43) der Mikrokapseln eine zweite Substanz enthält.

16. Flexibles Material nach Anspruch 15, wobei die erste Substanz und die zweite Substanz verschiedene Materialeigenschaften aufweisen.

17. Flexibles Material nach Anspruch 16, wobei die verschiedenen Materialeigenschaften aus wenigstens Duft, Schmelzpunkt, Viskosität, physikalischem Zustand, Farbe, Geschmack, chemischer Zusammensetzung und Struktur besteht.

18. Flexibles Material nach Anspruch 15, wobei der erste Teil von Mikrokapseln (42) mit der ersten Substanz derart in einem Gebiet des flexiblen Materials gruppiert ist, dass die Mittel zur Steuerung des Stromes örtlich das Gebiet erhitzen und die Freigabe der ersten Substanz ermöglichen.

19. Flexibles Material nach Anspruch 16, wobei die Mittel zur Steuerung des Stromes (5) eine örtliche Erhitzung des ersten (42) oder des zweiten (43) Teils der Mikrokapseln und kontrollierbar die Freigabe der ersten und zweiten Substanz ermöglichen.

20. Flexibles Material nach Anspruch 16, wobei die Mittel zur Steuerung des Stromes (5) eine örtliche Erhitzung des ersten (42) und des zweiten (43) Teils der Mikrokapseln ermöglichen und kontrollierbar die Freigabe eines Teils der ersten und der zweiten Substanz ermöglichen.

21. Flexibles Material nach Anspruch 15, wobei der erste Teil von Mikrokapseln (42) einen anderen Schmelzpunkt hat als der zweite Teil (43) von Mikrokapseln.

22. Flexibles Material nach Anspruch 21, wobei die Mittel zur Steuerung des Stromes (5) weiterhin Mittel aufweisen zur örtlichen Erhitzung des ersten (42) und des zweiten (43) Teils der Mikrokapseln, so dass nur der erste Teil (42) der Mikrokapseln zerrissen und die Substanz, die sie enthalten, freigegeben wird.

23. Flexibles Material, das Folgendes umfasst:
- verflochtene Fasern (20),
- Mittel zum Zuführen eines Stromes (23) und zum Erzeugen einer örtlichen Erhitzung zwischen den Fasern (20),
- wenigstens eine Substanz auf oder in den Fasern und den Mitteln zum Zuführen eines Stromes, die durch die örtliche Erhitzung, erzeugt durch selektive Erhitzung der Mittel zum Zuführen eines Stromes (23), verdampft, und
- Mittel zur Steuerung des Stromes (5) durch die Mittel zum Erzeugen örtlicher Erhitzung.

24. Verfahren zur kontrollierbaren Freigabe eines Substrats in einem flexiblen Material, wobei dieses Verfahren Folgendes umfasst:
- das Integrieren von Fasern (20) und Mitteln zum Zuführen eines Stromes (23) und zum Erzeugen örtlicher Erhitzung zwischen den Fasern (20),
- das Bilden wenigstens einer Mikrokapsel mit einer Substanz,
- das Einverleiben der wenigstens einen Mikrokapsel über oder in die integrierten Fasern (20) und der Mittel zum Zuführen eines Stromes (23),
- das selektive Erhitzen der wenigstens einen Mikrokapsel,
- das Zerreisen der wenigstens eine Mikrokapsel, und
- das Freigeben der genannten Substanz.

25. Verfahren zur kontrollierbaren Freigabe einer Substanz in einem flexiblen Material, wobei dieses Verfahren Folgendes umfasst:
- das Intergieren von Fasern (20) und Mitteln zum Zuführen eines Stromes (23) und zum Erzeugen örtlicher Erhitzung zwischen den Fasern (20);
- das Bilden einer Substanz über oder in den integrierten Fasern (20) und Mitteln zum Zuführen eines Stromes (23),
- das selektive Erhitzen der Substanz, und
- das Verdampfen der genannten Substanz.

## Revendications

1. Matériau souple comprenant :
des fibres entrelacées (20);
des moyens pour faire circuler un courant (23) et générer un chauffage localisé dispersé entre les fibres (20);
au moins une microcapsule située sur ou dans les fibres entrelacées (20) et les moyens pour faire circuler un courant (23), contenant une substance et libérant ladite substance suite au bris dû à un chauffage localisé généré en chauffant sélectivement les moyens pour faire circuler un courant (23); et
un moyen pour commander le courant (5) qui circule au travers des moyens pour faire circuler un courant (23), pour permettre un chauffage localisé qui peut être commandé.

2. Matériau souple selon la revendication 1, dans lequel les moyens pour faire circuler un courant (23) et générer un chauffage localisé comprennent en outre des fibres conductrices.

3. Matériau souple selon la revendication 1, dans lequel les moyens pour faire circuler un courant (23) et générer un chauffage localisé comprennent en outre une encre conductrice.

4. Matériau souple selon la revendication 1, dans lequel la au moins une microcapsule comprend en outre un polymère thermoplastique.

5. Matériau souple selon la revendication 4, dans lequel le polymère thermoplastique forme la au moins une microcapsule qui contient la substance à libérer.

6. Matériau souple selon la revendication 1, dans lequel la au moins une microcapsule libère la substance en atteignant son point de fusion.

7. Matériau souple selon la revendication 6, dans lequel la substance a un point de vaporisation inférieur au point de fusion de la au moins une microcapsule.

8. Matériau souple selon la revendication 1, dans lequel la substance est composée d'au moins un parmi une huile, un liquide et un matériau solide.

9. Matériau flexible selon la revendication 1, dans lequel la substance génère un parfum par libération dans l'environnement ambiant.

10. Matériau souple selon la revendication 1, dans lequel le moyen pour commander le courant (5) comprend en outre une alimentation (4) et un sélecteur de chemin de courant (5).

11. Matériau souple selon la revendication 10, dans lequel le moyen pour commander le courant (5) comprend en outre au moins un capteur programmable qui détermine quand activer et désactiver les moyens pour faire circuler un courant.

12. Matériau souple selon la revendication 11, dans lequel le au moins un capteur programmable détecte quand un nombre prédéterminé de microcapsules sont brisées.

13. Matériau souple selon la revendication 10, dans lequel le moyen pour commander le courant comprend en outre un compteur de temps.

14. Matériau souple selon la revendication 13, dans lequel le compteur de temps détermine quand désactiver les moyens pour faire circuler un courant (23) sur base du point de fusion de la au moins une microcapsule, du nombre de microcapsules et des propriétés de matériau de la substance contenue dans la au moins une microcapsule.

15. Matériau souple selon la revendication 1, comprenant en outre des microcapsules multiples, parmi lesquelles une première partie (42) des microcapsules contient une première substance et une deuxième partie (43) des microcapsules contient une deuxième substance.

16. Matériau souple selon la revendication 15, dans lequel la première substance et la deuxième substance ont des propriétés de matériau différentes.

17. Matériau souple selon la revendication 16, dans lequel les propriétés de matériau différentes sont au moins une parmi les parfum, point de fusion, viscosité, état physique, couleur, saveur, composition chimique et texture.

18. Matériau souple selon la revendication 15, dans lequel la première partie des microcapsules (42) contenant la première substance est groupée sur ou dans une zone du matériau souple de façon que les moyens pour commander le courant chauffent localement la zone et permettent la libération de la première substance.

19. Matériau souple selon la revendication 15, dans lequel le moyen pour commander le courant (5) permet un chauffage local de soit la première (42) soit la deuxième (43) partie des microcapsules et permet de façon commandée la libération de la première ou de la deuxième substance.

20. Matériau souple selon la revendication 16, dans lequel le moyen pour commander le courant (5) permet un chauffage local des première (42) et deuxième (43) parties des microcapsules et permet de façon contrôlée la libération d'une partie de la première et de la deuxième substance.

21. Matériau souple selon la revendication 15, dans lequel la première partie de microcapsules (42) a un point de fusion différent de la deuxième partie (43) de microcapsules.

22. Matériau souple selon la revendication 21, dans lequel le moyen pour commander le courant (5) comprend en outre un moyen pour chauffer localement les première (42) et deuxième (43) parties de microcapsules de façon que seule la première partie de microcapsules se brise et libère la substance qu'elles contiennent.

23. Matériau souple comprenant :
des fibres entrelacées (20);
des moyens pour faire circuler un courant (23) et générer un chauffage localisé dispersé entre les fibres (20);
au moins une substance, située sur ou dans les fibres et les moyens pour faire circuler un courant, qui s'évapore du fait d'un chauffage localisé généré en chauffant de façon sélective les moyens pour faire circuler un courant (23); et
un moyen pour commander le courant (5) qui circule au travers des moyens pour générer un chauffage localisé.

24. Procédé pour libérer de façon qui peut être commandée une substance contenue dans un matériau souple, qui comprend :
le fait d'intégrer des fibres (20) et des moyens pour faire circuler un courant (23) et générer un chauffage localisé dispersé entre les fibres (20);
le fait de former au moins une microcapsule qui contient une substance;
le fait d'incorporer la au moins une microcapsule sur ou dans les fibres (20) et les moyens pour faire circuler un courant (23) intégrés;
le fait de chauffer sélectivement la au moins une microcapsule;
le fait de briser la au moins une microcapsule; et
le fait de libérer ladite substance.

25. Procédé pour libérer de façon qui peut être commandée une substance contenue dans un matériau souple, qui comprend :
le fait d'intégrer des fibres (20) et des moyens pour faire circuler un courant (23) et générer un chauffage localisé dispersé entre les fibres (20);
le fait de former une substance sur ou dans les fibres (20) et les moyens pour faire circuler un courant (23) intégrés;
le fait de chauffer la substance de façon sélective; et
le fait de libérer ladite substance.
